# EUROPEAN PATENT APPLICATION

(11) **EP 1 418 524 A2**
(43) Date of publication of application: **12.05.2004**
(21) Application number: 03256764.6
(22) Date of filing: 27.10.2003
(51) Int. Cl.: G06F 19/00

(54) **Pharmaceutical supply system**

(30) Priority: 29.10.2002 US 283547
(71) Applicant: Hewlett-Packard Development Company, L.P., Houston, TX 77070 (US)
(72) Inventor: Greeven, John C., Corvallis, OR 97330 (US); Valley, Jeffrey M., Corvallis, OR 97330 (US)
(74) Representative: Jackson, Richard Eric

(57) **Abstract**

A pharmaceutical supply method is provided which includes receiving a request to charge a delivery appliance with a requested pharmaceutical, producing a first electronic pharmaceutical identifier which identifies the requested pharmaceutical, reading an identification tag associated with an actual pharmaceutical to produce a second electronic pharmaceutical identifier which identifies the actual pharmaceutical, and comparing the first electronic pharmaceutical identifier with the second electronic pharmaceutical identifier. Where the first electronic pharmaceutical identifier matches the second electronic pharmaceutical identifier, the delivery appliance may be charged with the actual pharmaceutical.

## Description

### BACKGROUND

Each year, a significant number of pharmaceuticals are inaccurately dispensed or delivered to patients in need. These inaccurately dispensed or delivered pharmaceuticals may result from difficult-to-read handwriting on prescriptions, similar pharmaceutical names, and/or similar appearance of distinct pharmaceuticals or their packaging. Errors also may be the result of similarities between patient names, leading to confusion either at the time of providing the pharmaceutical to the patient, or at the time of patient use. Still other errors may result from confusing instructions, or intentional variations by a patient to a dosing regimen prescribed by the physician, pharmacist or manufacturer of the pharmaceutical. Such errors may have the potential to cause severe injury to the patient. Accordingly, it would be desirable to provide a pharmaceutical supply system whereby pharmaceuticals may be more reliably dispensed to patients.

### SUMMARY

A pharmaceutical supply method is provided which includes receiving a request to charge a delivery appliance with a requested pharmaceutical, producing a first electronic pharmaceutical identifier which identifies the requested pharmaceutical, reading an identification tag associated with an actual pharmaceutical to produce a second electronic pharmaceutical identifier which identifies the actual pharmaceutical, and comparing the first electronic pharmaceutical identifier with the second electronic pharmaceutical identifier. Where the first electronic pharmaceutical identifier matches the second electronic pharmaceutical identifier, the delivery appliance may be charged with the actual pharmaceutical.

### BRIEF DESCRIPTION OF THE DRAWINGS

Fig. 1 is a somewhat schematic representation of a system configured to verify that a pharmaceutical is accurately supplied according to an embodiment of the present invention.
Fig. 2 is a schematic representation of a delivery appliance according to an embodiment of the present invention.
Fig. 3 is a flow chart illustrating a method of verifying that a prescription has been filled and dispensed accurately, according to an embodiment of the present invention.

### DETAILED DESCRIPTION

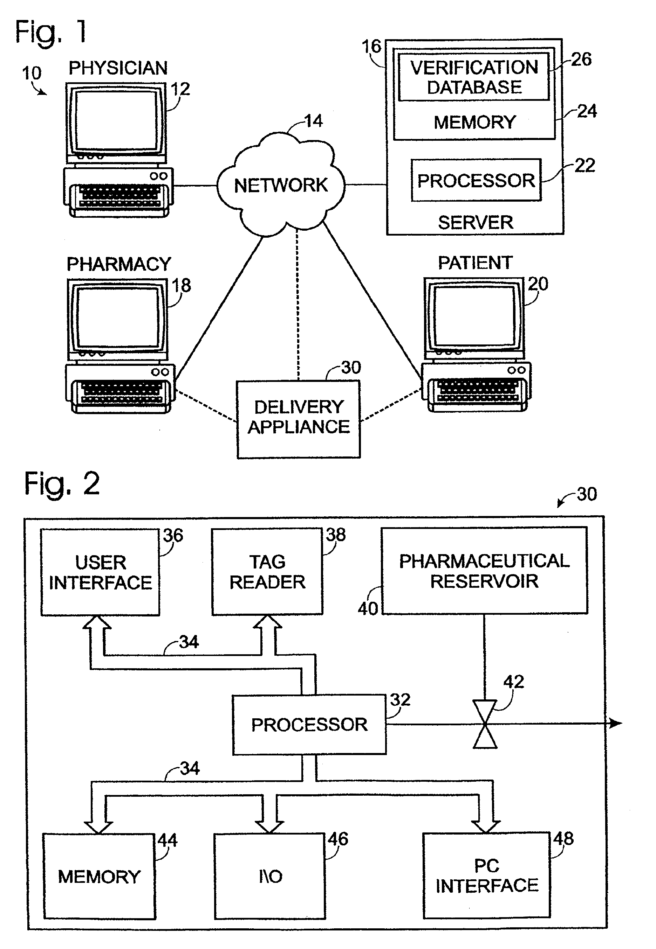

Referring initially to Fig. 1, a pharmaceutical supply system according to an embodiment of the present invention is shown, the system being indicated generally at 10. As illustrated, system 10 may include a physician terminal 12 connected, via a network communication link 14, to a server 16. System 10 may also include a pharmacy terminal 18 and a patient terminal 20, each of which may also be connected to server 16 via network communications link 14.

Server 16, in turn, may include a processor 22 operatively connected to memory 24, which may store a verification source, such as verification database 26, for use in verifying proper distribution of pharmaceuticals via a delivery appliance 30, as will be described further below. As indicated, delivery appliance 30 may be configured to connect to one or more of terminals 12, 18, and 20, or may connect directly to network communications link 14 for access to the terminals and/or server 16.

In accordance with the present embodiment, physician terminal 12 may communicate prescription records and other data to server 16 for storage. Each prescription record may be an electronic copy of a particular prescription for a particular patient, and thus may be characterized as a unique prescription request (typically having a unique request identifier). The prescription record may contain information from the physician's records, including patient-specific identification information such as name, address, age, sex, height, weight, known allergies, a patient identifying image, etc. The prescription record also may include patient-specific prescription information such as a pharmaceutical identifier, and a dosing regimen. This information may be stored on physician terminal 12, or, as noted above, communicated to server 16, or any of the designated terminals. In some instances, only selected portions of the information from the physician's records may be communicated to server 16.

Network communications link 14 typically connects physician terminal 12, pharmacy terminal 18 and patient terminal 20 to server 16. The network communications link may be any type of network capable of communicating data. For example, network communications link 14 may be a local area network (LAN), a wide area network (WAN), or any combination of these. Network communications link 14 may use any suitable network architecture enabling terminals 12, 18, and 20 to exchange data securely with server 16.

One embodiment of the present invention, as shown in Fig. 1, is implemented using a wide area network (WAN) as the network communication link. Pharmacy terminal 18 may be linked to server 16 through the WAN. Physician terminal 12 and patient terminal 20 also may be linked to server 16 through the WAN. Delivery appliance 30 may be linked to any of the aforementioned terminals, or directly to server 16 through the WAN. Each of terminals 12, 18, and 20, as well as delivery appliance 30, may securely transmit and receive data to and from server 16 through the WAN.

Server 16 may be any suitable network server computer configured to store data and to be accessed from a proximate or remote terminal or terminals. A single computer or a set of networked computers may function as the server. Access to server 16 may be through a user interface on the server itself, or may be through one or more of the designated terminals.

Processor 22 of server 16 may be configured to accommodate verification of proper distribution of a pharmaceutical using a prescription record from verification database 26. As indicated, verification database 26 may be stored in memory 24 of server 16, and/or may be stored in memory of one or more of the designated terminals. Additionally, the verification database may be multiple databases, stored on multiple servers, at proximate or disparate locations. For example, each of a plurality of physicians may maintain a physician-specific verification database having patient-specific identification and/or prescription information.

In one embodiment, verification database 26 stores prescription records from a physician or physicians for use in verifying patient identification and prescription information presented to a pharmacist. Accordingly, a physician may write a prescription using a personal digital assistant (PDA) configured to transmit the prescription information to physician terminal 12. Software on physician terminal 12, in turn, may transmit a prescription record to server 16 via network communications link 14 to be stored in verification database 26. Upon receiving a prescription request, a pharmacist may then check the authenticity of the prescription request by accessing the verification database and searching for a prescription request that matches the received request. Where a match is found (for example, by matching electronic request identifiers in the recorded request and the received request), information may be verified, including patient name, pharmaceutical name, dosing regimen, etc.

As alluded to above, a match may be considered to occur when a unique identifier code from the received request is found in a corresponding field of a prescription record. Alternatively, or additionally, a match may be considered to occur when a predetermined minimum number or fields in a received electronic request match corresponding fields in a recorded electronic request in a prescription record of the verification database. It should be understood that some fields may be more heavily weighted than others when searching for a match. For example, a match may be found if the name of the patient and the pharmaceutical of the request are the same in the received and recorded electronic requests.

Information in verification database 26 may be kept current as new prescriptions are written. A proprietor of system 10 thus may direct updating of the database with prescriptions by having physicians or medical service providers create electronic prescription records at the time prescriptions are written, and transmit these electronic prescription records to server 16 for storage on verification database 26.

In one embodiment, the verification database may be stored in a specialized server. In this embodiment, the prescription request that the patient gives to the pharmacist may include a code that enables the pharmacist to remotely access the verification database stored on the specialized server. This embodiment may enable a physician to maintain a patient database having limited access.

In another embodiment, the verification database may be stored on physician terminal 12. In this embodiment, the prescription request that the patient gives to the pharmacist may include a code or address that enables the pharmacist to remotely access the verification database stored on physician terminal 12.

Pharmacy terminal 18 typically is located at a pharmacy in order to aid the pharmacist in record-keeping, in communicating with server 16 and/or the other terminals, and in verifying pharmaceutical transactions such as filling of prescriptions. A typical pharmacy terminal may be a computer terminal including a processor, memory, a user interface, etc. The pharmacy terminal thus typically permits a pharmacist to access server 16, and to retrieve information stored on the server for use in verification of proper distribution of a pharmaceutical, and/or in production of a custom label for a pharmaceutical. The pharmacy terminal also may be used to direct charging of delivery appliance 30, to verify proper charging of the delivery appliance, and/or to direct operation of such delivery appliance in accordance with physician, pharmacist, and/or pharmaceutical manufacturer dosing directives (e.g., via programming of the delivery appliance).

Patient terminal 20 also typically may be a computer terminal configured to interact with delivery appliance 30, and to communicate with other terminals and server 16. The patient thus may use the patient terminal to produce a custom label, to verify contents of the delivery appliance, to request dispensing of a dose of the pharmaceutical and/or to request that a prescription be filled by an online pharmacy.

It will be understood that while terminals 12, 18 and 20 are illustrated and described as computer terminals each may also include a plurality of networked computers. For example, a physician or pharmacy terminal as described herein may actually include multiple computer terminals connected to a local computer network.

Furthermore, each terminal may include an identifying network address for communicating with server 16. In this embodiment a terminal may not be able to access server 16 without authorization. Authorization may require a pre-approved user name and/or password, or may require submission of the pharmacist's licensing data (for a pharmacy terminal). Similarly, physician terminal 12 and patient terminal 20 may require an identifying network address or similar authorization to access server 16.

Delivery appliance 30 may be configured to connect to network communications link 14, either directly, or via another terminal, as illustrated in Fig. 1. Delivery appliance 30 may be used to verify the patient identity, pharmaceutical identity and/or dosing regimen of a pharmaceutical when the pharmacist or patient charges the delivery appliance with a pharmaceutical. Similar verification may be achieved when the patient requests delivery of a pharmaceutical dose.

Fig. 2, illustrates, schematically, delivery appliance 30. As indicated, delivery appliance 30 may include a controlling processor 32, which may take the form of a microcontroller, a microprocessor, a digital signal processor (DSP), etc. The controlling processor typically is operatively coupled with other components of the delivery appliance that may include, but are not limited to, a data bus 34, a user interface 36, a tag reader 38, a reservoir 40, a dispensing mechanism (e.g., control valve) 42, memory 44, input/output 46 and a PC interface 48. Controlling processor 32 of delivery appliance 30 typically connects to user interface 36, tag reader 38 and other components of the delivery appliance through data bus 34.

Controlling processor 32 may interact with a user via user interface 36. The user interface may include a display screen, such as a liquid crystal display, or a thin film transistor screen. The user interface may have touch-screen capability, or it may include an associated keypad. The user interface typically enables the user to enter information and make dispensing requests, as will be explained in more detail below.

Tag reader 38 typically is configured to read a tag on or in connection with the pharmaceutical selected to fill reservoir 40. The tag may include pharmaceutical information such as the identity of the pharmaceutical, possible drug-to-drug interactions, maximum dosages, etc. In one embodiment, tag reader 38 may be configured to read a smart-card-type tag on a container of a prescription pharmaceutical. Alternatively, tag reader 38 may be an optical reader for scanning a bar code or similar identifying indicia. In yet another embodiment, tag reader 38 may be configured to read electronic tags such as a serial memory device encoded with the aforementioned information. It will be understood, that any suitable method for data communication may be used to enable the tag reader to obtain information about the pharmaceutical selected to charge reservoir 40.

Tag reader 38 thus may read pharmaceutical identification information from the tag of a selected pharmaceutical, and record this selected pharmaceutical identification information in memory 44 of delivery appliance 30. Correspondingly, I/O port 46 and/or PC interface 48 (described below) may be used by processor 32 to access a record including pharmaceutical identification information identifying a requested pharmaceutical. This requested pharmaceutical identification information also may be stored in memory 44 of delivery appliance 30. The processor thus may compare the selected pharmaceutical identification information with the requested pharmaceutical identification information to verify that they match, and thus that the reservoir will be, is being, or has been properly charged. Where they do match, proper charging of the reservoir may be permitted (or confirmed). Where they do not match, a charge error prevention mechanism (such as a display of user interface 36) may be triggered to discourage improper charging of the reservoir (as by notifying the user that the reservoir is being (or has been) improperly charged).

As used herein, pharmaceutical includes any controlled or medicinal substances (such as tablets, liquids, gases, etc.), intended for administration to a patient according to a treatment regimen, or by prescription of a medical services professional. Medical services professionals include a physicians, nurse practitioners, etc. Reservoir 40 may also store dispensable supplies, such as syringes, reagent test strips (for blood glucose testing, for example), antihistamine tablets and the like, also according to some prescribed treatment regiment. It will be understood that the delivery appliance typically is configured to dispense a pharmaceutical that might be dispensed to, or used by, a patient. It also will be understood that, although delivery appliance 30 is primarily discussed herein with reference to its uses for prescription pharmaceuticals, it may be used to dispense non-prescription consumables (such as, vitamins, dietary supplements, etc.). Accordingly, the system may be used to verify proper charging of the delivery appliance with such prescription and non-prescription consumables, and to direct delivery of such consumables in accordance with a desired dispensing regimen.

Delivery appliance 30 may be configured to accommodate a variety of differing types of reservoirs, depending on the type of pharmaceutical or consumable being dispensed. For example, an inhaled prescription pharmaceutical may be stored in a different type of reservoir than pills or capsules. In some embodiments, delivery appliance 30 may be configured to store and dispense a plurality of pharmaceuticals. In these embodiments the delivery appliance may be configured to employ plural reservoirs. Reservoir 40 may be opened and closed by processor 32 through commands to a gate, valve or other dispensing mechanism 42. In embodiments of the delivery appliance that include plural reservoirs, a plurality of independent control gates, valves or dispensing mechanisms may be employed.

A dispensing regimen (which typically is a schedule or circumstances according to which a prescription pharmaceutical is taken by, or administered to, a patient) may be downloaded from a physician terminal, a pharmacist terminal or a verification database (or entered manually through the user interface), and stored as a set of instructions or parameters in memory 44. By following the stored set of instructions, the delivery appliance may be automatically directed to reliably dispense pharmaceuticals according to a medical service provider's prescribed dispensing regimen.

Delivery appliance 30 may include an input/output (I/O) port 46 and a PC interface 48. Both I/O port 46 and PC interface 48 may be types of data network interfaces configured to permit the delivery appliance to communicate with other devices. The I/O port may be wireless or wired, and any suitably reliable and secure communications standard may be used to transfer data.

The I/O port may be configured to connect directly to a network such that the delivery appliance may itself function as a terminal on the network. Similarly, the PC interface 48 may be used to connect drug-delivery appliance 30 with a personal computer terminal. PC interface 48 allows a terminal to interact with the delivery appliance. The PC interface may be similar to the interface between a personal digital assistant (PDA) and a desktop personal computer in that data may be exchanged, and the desktop personal computer may act as host for loading software and synchronizing data with the PDA, or in this case, the delivery appliance. The PC interface connectivity may provide faster data transport than the I/O interface and may enable additional functions, such as providing power to operate the delivery appliance and/or recharge a battery of the delivery appliance.

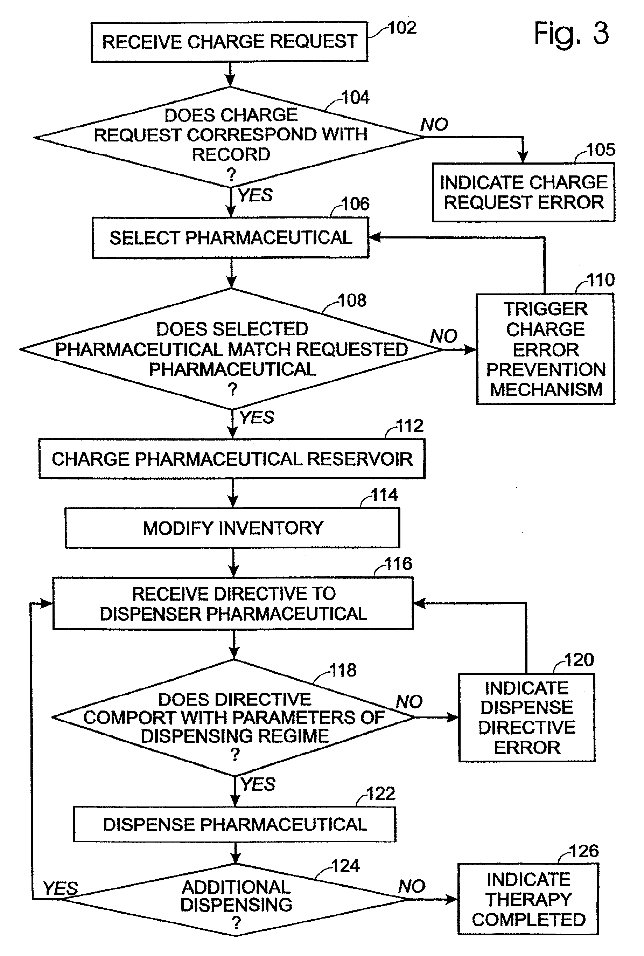

Fig. 3 is a flow chart, which schematically illustrates, at 100, a method of using system 10 to verify that pharmaceuticals are accurately dispensed according to a desired dispensing regimen. Method 100 includes receiving a request to provide a pharmaceutical as by charging a reservoir of a delivery appliance with the pharmaceutical, as shown at 102. Such request may be pursuant to a physician's prescription, or based on a dispensing regimen selected by the patient or pharmacist within guidelines set by a physician or manufacturer of the pharmaceutical.

Verifying the authenticity and accuracy of such a request, referred to herein as a charge request, may be accomplished by the delivery appliance accessing a verification database to determine whether the request corresponds with a record in the verification database, as shown at 104. For purposes of illustration, it will be appreciated that the charge request may take the form of a prescription request, which may be compared to prescription records in the verification database.

A pharmacist thus may enter a prescription request into a pharmacy terminal to produce an electronic request corresponding to the prescription request. The delivery appliance similarly may receive the prescription request through a user interface, or by downloading the prescription request from a terminal or other device, via either the I/O port or the PC interface, and may produce the electronic request corresponding to the prescription request. In either case, such an electronic request is referred to herein as a received electronic request. The verification database, in turn, may be considered to include prescription records corresponding to actual prescriptions, and thus may provide an electronic request corresponding to the prescription record. Such electronic requests are referred to herein as recorded electronic requests.

The verification database thus may be searched to determine whether corresponding electronic requests exist by sending a verification message (including, for example, an electronic identifier of the prescription request) to the server via a network interface, such as the I/O port, the PC interface, or similar communication interface on the pharmacist terminal. In response to the verification message, the server may search for a matching electronic identifier of a prescription record in the verification database, and may send a verification response indicative of whether such a matching electronic identifier was found.

The verification message sent to the verification database may include a prescription request with patient identity information, pharmaceutical identity information, and/or some other electronic identifier configured to uniquely identify a prescription request. The electronic identifier may be any code, marker, or other data that uniquely identifies the prescription request. As noted above, such an identifier may facilitate the matching of prescription request to prescription record in the verification database.

If a matching record is found (for example, by the presence of a matching electronic request in a prescription record), the verification response may indicate that the received electronic request is authentic, and a pharmaceutical may be selected for use in charging the delivery appliance reservoir, as shown at 106. The verification response may include an electronic pharmaceutical identifier identifying the requested pharmaceutical, as derived, for example, from the prescription record (or verified prescription request).

The verification response also may include instructions to the delivery appliance, thereby setting parameters for dispensing the pharmaceutical according to a prescribed or desired dispensing regimen. These instructions, in effect, may control the dispensing of the pharmaceutical from the reservoir. For example, the instructions may set an interval between permitted doses of the pharmaceutical, permitting operation of the dispensing mechanism only in accordance with these intervals.

If a matching record is not found, the verification response may indicate an error in the charge request to the pharmacist, as shown at 105. The verification response also may inform a medical service provider of an error in the prescription request, and/or may alert the medical service provider of an attempt to alter a prescription request.

As indicated, the tag reader of the delivery appliance may be configured to read a pharmaceutical identification tag associated with the pharmaceutical selected to charge the reservoir, thereby producing an electronic identifier which identifies the pharmaceutical actually to be used. It should again be noted that other information may also be included on the tag, and read by the reader, including possible drug-to-drug interactions, lot number information, etc. Once a prescription request has been verified, the identity of the pharmaceutical actually selected to charge the pharmaceutical reservoir may be checked to ensure that it matches the pharmaceutical called for in the verified prescription request, as shown at 108.

If the selected pharmaceutical does not match the requested pharmaceutical (as determined, for example, by comparison of respective electronic pharmaceutical identifiers), charge error prevention mechanism may be triggered to discourage use of that pharmaceutical to charge the pharmaceutical reservoir, as indicated at 110. The charge error prevention mechanism may be a warning message on the user interface of the delivery appliance to discourage charging of the reservoir with the selected pharmaceutical, or an actual locking structure to prevent an incorrectly-selected pharmaceutical from being inserted in the pharmaceutical reservoir. If the selected pharmaceutical does match the requested pharmaceutical, then the selected pharmaceutical may be provided (e.g. the pharmaceutical reservoir may be charged, as indicated at 112, with the selected pharmaceutical).

In another embodiment, the verification response may be sent by the delivery appliance after the pharmaceutical reservoir has already been filled. If the selected pharmaceutical does not match the requested pharmaceutical, the verification response from the server may direct the delivery appliance to prevent dispensing of the requested pharmaceutical.

An inventory, or count of the doses, of the pharmaceutical remaining in the pharmaceutical reservoir also may be modified and stored in the memory of the delivery appliance, as indicated at 114. The initial inventory may be read from the tag, downloaded from the pharmacist terminal, or entered manually. Once the delivery appliance has been charged and the therapy regimen has been stored as a set of parameters in memory, the delivery appliance may receive directives from the patient to dispense the pharmaceutical from the pharmaceutical reservoir, as indicated at 116.

Upon receipt of a directive to dispense the pharmaceutical from the pharmaceutical reservoir, the processor may access the delivery appliance memory to verify that the dispense directive comports with the parameters of the prescribed dispensing regimen, as indicated at 118. For example, the processor may check to determine how long it has been since the last dose was administered before directing the dispensing mechanism to dispense another dose of the pharmaceutical. If the dispense directive does not comport with the parameters of the prescribed dispensing regimen, the processor may indicate a dispense directive error, for example, by displaying an error message on the user interface of the delivery appliance, as indicated at 120. The processor also may deny such a directive. Furthermore, the processor may check the information read from the tag associated with the contents of the reservoir for an expiration date. If the contents of the reservoir have expired, the processor may indicate an error and/or prevent additional doses from being dispensed.

It may also be desirable to notify the patient's medical service provider if one or more dispense requests are received by the delivery appliance that do not comport with the prescribed regimen. This information may be sent to the verification database by the delivery appliance, or may be communicated to the physician or pharmacist. The delivery appliance may automatically connect to the network via a wireless connection to communicate this information to the medical services provider, or may transmit this data when it is connected to a terminal.

The identity of the patient making a dispense directive also may be verified at the time of such request using parameters stored in the memory of the delivery appliance. The prescription record may include patient identity data that may be stored in memory of the delivery appliance. The delivery appliance may thereafter require the patient making a dispense directive to prove their identity. This may be done through biometric fingerprinting or similar technology, through a password, through a keycard, or some other method for proving the identity of the patient making the request. If the dispense directive does comport with the parameters of the prescription record, the processor may command the dispensing mechanism to dispense the pharmaceutical from the reservoir, as indicated at 122.

After each dose is dispensed the processor may check the therapy regimen to determine if the regimen calls for another dose to be dispensed, as indicated at 124. If the parameters indicate that an additional dose is to be dispensed, the delivery appliance may be prepared to receive another directive to dispense the next dose of the pharmaceutical. If the therapy regimen has been completed the delivery appliance may transmit a message indicating that the therapy regimen has been completed, as indicated 126. The message also may be sent to the physician's terminal, the verification database of the server, and/or any other suitable terminal or server. The delivery appliance may transmit the message after automatically connecting to the network, or it may transmit the message when a patient manually causes the delivery appliance to connect to the network.

While an embodiment of the invention has been particularly shown and described, those skilled in the art will understand that many variations may be made therein without departing from the spirit and scope defined in the following claims. The present description thus should be understood to include all novel and non-obvious combinations of elements described herein, and claims may be presented in this or a later application to any novel and non-obvious combination of these elements. The foregoing embodiments are illustrative, and no single feature or element is essential to all possible combinations that may be claimed in this, or a later application. Where the claims recite "a" or "a first" element or the equivalent thereof, such claims should be understood to include incorporation of one or more such elements, neither requiring nor excluding two or more such elements.

## Claims

1. A pharmaceutical supply method comprising: receiving a request (102) to provide a pharmaceutical; producing a first electronic pharmaceutical identifier which identifies the requested pharmaceutical; reading an identification tag associated with an actual pharmaceutical to produce a second electronic pharmaceutical identifier which identifies the actual pharmaceutical; and comparing the first electronic pharmaceutical identifier (108) to the second electronic pharmaceutical identifier; where the first electronic pharmaceutical identifier matches the second electronic pharmaceutical identifier, providing the actual pharmaceutical.

2. The method of claim 1, wherein receiving the request to provide the pharmaceutical includes: receiving an electronic request identifier; and accessing a verification source to search prescription records (104) for a recorded electronic request which corresponds to the received electronic request, presence of such corresponding electronic request in the prescription records being indicative of authenticity of such request to provide a pharmaceutical.

3. The method of claim 2, wherein receiving an electronic request identifier includes at least one of optically reading a prescription and electronically reading a prescription.

4. The method of claim 1, wherein producing a first electronic pharmaceutical identifier includes accessing a verification source including such first electronic pharmaceutical identifier.

5. The method of claim 1, further comprising: charging a delivery appliance (112) with the actual pharmaceutical; and dispensing the pharmaceutical (122) from the delivery appliance according to predetermined dispensing parameters upon determining that the first electronic pharmaceutical identifier matches the second electronic pharmaceutical identifier.

6. The method of claim 1, further comprising: receiving a directive to dispense (116) a dose of the pharmaceutical; checking to determine (118) whether the interval of time between dispensing doses has elapsed; wherein, if the interval of time between (122) dispensing doses has elapsed, dispensing a dose of the pharmaceutical; recording an inventory of pharmaceutical stored in the delivery appliance; and modifying the inventory as each dose of pharmaceutical is dispensed to track such inventory.

7. A pharmaceutical delivery appliance (30) for use in delivering a pharmaceutical requested for delivery to a patient, the appliance comprising: a processor (32) configured to access requested pharmaceutical identification information, and the processor (32) is further configured to receive an electronic request to charge the pharmaceutical delivery appliance (30) with a prescribed pharmaceutical, and is configured to access a verification source (26) to search prescription records for a recorded electronic request which corresponds to the received electronic request, presence of such a corresponding electronic request in the prescription records being indicative of authenticity of the received electronic request; a tag reader (38) configured to read selected pharmaceutical identification information from an identification tag associated with a pharmaceutical selected for use in charging the delivery appliance; and an error prevention mechanism configured to discourage charging of the delivery appliance where the requested pharmaceutical identification information does not correspond with the selected pharmaceutical identification information.

8. The pharmaceutical delivery appliance (30) of claim 7, which further comprises a dispenser (42) configured to dispense pharmaceuticals from the delivery appliance (30) according to predetermined dispensing parameters upon determining that the requested pharmaceutical identification information corresponds with the selected pharmaceutical identification information, wherein the predetermined dispensing parameters determine an interval of time between dispensing doses of the pharmaceutical.

9. A pharmaceutical delivery appliance (30) comprising: a processor (32) configured to access a prescription record including an electronic identifier which identifies a prescribed pharmaceutical, wherein the processor (32) is further configured to receive a prescription request from a user, and to access a verification source (26) to authenticate the received prescription request, the processor (32) being adapted to search the verification source (26) for a recorded prescription request which corresponds to the received prescription request, presence of such corresponding prescription requests being indicative of authenticity of the received prescription request; a tag reader (38) coupled with the processor (32) to read an electronic identifier from an identification tag associated with an actual pharmaceutical and communicate such actual pharmaceutical electronic identifier to the processor (32); a reservoir (40) configured to accept the prescribed pharmaceutical; and a dispensing mechanism (42) coupled with the processor (32) and the reservoir(40), the dispensing mechanism (42) being responsive to the processor (32), and configured to dispense the pharmaceutical from the reservoir (40) upon identifying a match between the electronic identifier which identifies the prescribed pharmaceutical and the electronic identifier from the identification tag associated with the actual pharmaceutical in accordance with predetermined dispensing parameters of the prescription record.

10. A pharmaceutical delivery appliance (30) for use in delivering a pharmaceutical requested for delivery to a patient, the appliance comprising: processor means (32) for accessing requested pharmaceutical identification information; tag reader means (38) for reading selected pharmaceutical identification information from an identification tag associated with a pharmaceutical selected for use in charging the delivery appliance; and error prevention means for discouraging charge of the delivery appliance where the requested pharmaceutical identification information does not correspond with the selected pharmaceutical identification information, wherein the error prevention means includes a display configured to indicate an inaccurate pharmaceutical selection.
